Europäisches Patentamt

(19) European Patent Office

Office européen des brevets

(11) Numéro de publication: **0 276 618**
**A1**

(12) **DEMANDE DE BREVET EUROPEEN**

(21) Numéro de dépôt: **87403011.7**

(22) Date de dépôt: **30.12.87**

(51) Int. Cl.⁴: **A61B 1/30** , A61B 5/00

(30) Priorité: **30.12.86 FR 8618337**

(43) Date de publication de la demande:
**03.08.88 Bulletin 88/31**

(84) Etats contractants désignés:
**DE ES GB IT**

(71) Demandeur: **Abensour, David**
**27, Rue Hermite**
**F-54000 Nancy(FR)**

Demandeur: **Cinqualbre, Jean**
**30, Rue Hermite**
**F-54000 Nancy(FR)**

(72) Inventeur: **Karcher, Gilles**
**2, Rue Lafayette**
**F-54000 Nancy(FR)**
Inventeur: **Amor, Max**
**9, Square de Liège**
**F-54500 Vandoeuvre(FR)**
Inventeur: **Abensour, David**
**27, Rue Hermite**
**F-54000 Nancy(FR)**
Inventeur: **Cinqualbre, Jean**
**30, Rue Hermite**
**F-54000 Nancy(FR)**

(74) Mandataire: **Laget, Jean-Loup et al**
**Cabinet Pierre Loyer 77, rue Boissière**
**F-75116 Paris(FR)**

(54) **Amnioscope.**

㊾ Un appareil 2 de mesure, contenant une source
de lumière et un analyseur spectrométrique, est relié
par un câble 3 à fibres optiques, à une sonde 1
remplaçable de faible diamètre, susceptible d'être
utilisée même lorsque le col utérin est dit "fermé".

**Fig. 1**

## AMNIOSCOPE

L'invention concerne un amnioscope, c'est-à-dire un appareil d'examen du liquide amniotique in-utero.

Un liquide amniotique, contenu dans la poche des eaux, a normalement une teinte claire, dite "eau de roche", ou légèrement opalescente. Dans certaines circonstances pathologiques, le foetus peut émettre du méconium, qui fait perdre au liquide amniotique ses propriétés bactériostatiques et entraîne ainsi un risque septique majeur pour l'enfant, imposant une extraction rapide.

Il est connu de procéder à un examen du liquide amniotique au moyen d'un amnioscope. Les amnioscopes classiques sont constitués essentiellement par une barre transparente qui permet un examen oculaire, par le practicien, de la couleur du liquide amniotique. L'utilisation des ces amnioscopes présente deux inconvénients. Tout d'abord, la barre transparente a un diamètre d'environ 2cm, ce qui limite son utilisation aux dernières semaines de la grossesse, lorsque le col utérin est déjà partiellement dilaté.

Ensuite, l'observation de la couleur du liquide amniotique est purement visuelle, et donc subjective et le risque d'erreur d'interprétation est estimé à 30% environ.

Un but de la présente invention est de proposer un amnioscope utilisable pendant les deux derniers mois, environ, de la grossesse, sans pour autant être traumatisant pour le col utérin.

Un autre but de la présente invention est de proposer un amnioscope permettant une interprétation objective des résultats, réduisant ainsi les risques d'erreur.

L'invention a pour objet un amnioscope à sonde conductrice de lumière pour l'examen du liquide amnioptique in-utéro, caractérisé en ce qu'il comporte : une sonde remplaçable de faible diamètre contenant des fibres optiques pour la transmission de la lumière d'éclairement du liquide amniotique et de la lumière diffusée par le liquide amniotique ; un appareil de mesure contenant une source de lumière de spectre défini et un analyseur spectroscopique ; et un câble à fibres optiques relié d'une part audit appareil de mesure et d'autre part à un connecteur portant la sonde.

Selon d'autres caractéristiques de l'invention :
- la sonde présente à son extrémité distale un embout transparent et souple, porté par une gaîne tubulaire ;
- dans la gaîne sont disposées au moins une fibre optique d'amenée de lumière à l'embout et au moins une fibre optique de transmission de la lumière diffusée par le liquide amniotique ;
- dans la gaîne est disposé un canal débouchant à l'extrémité distale de l'embout et susceptible d'amener du liquide de lavage ;
- le connecteur portant la sonde est relié par un tuyau à une source de liquide aseptique ;
- la sonde est recouverte d'une enveloppe de protection, déchirable par traction après mise en place de la sonde ;
- la source de lumière a un spectre défini pour contenir les longueurs d'onde des pigments dont la présence est recherchée dans le liquide amniotique, et correspondant au méconium, à la bilirubine et à l'hémoglobine ;
- le diamètre de la sonde est d'environ 5mm, ce qui autorise l'examen du liquide amniotique même lorsque le col utérin est dit "fermé",

D'autres caractéristiques de l'invention ressortent de la description qui suit faite avec référence au dessin annexé sur lequel on peut voir :

Figure 1 - un schéma d'ensemble de l'amnioscope selon l'invention ;

Figure 2 - une vue en coupe à échelle agrandie de l'extrémité distale de la sonde de l'amnioscope.

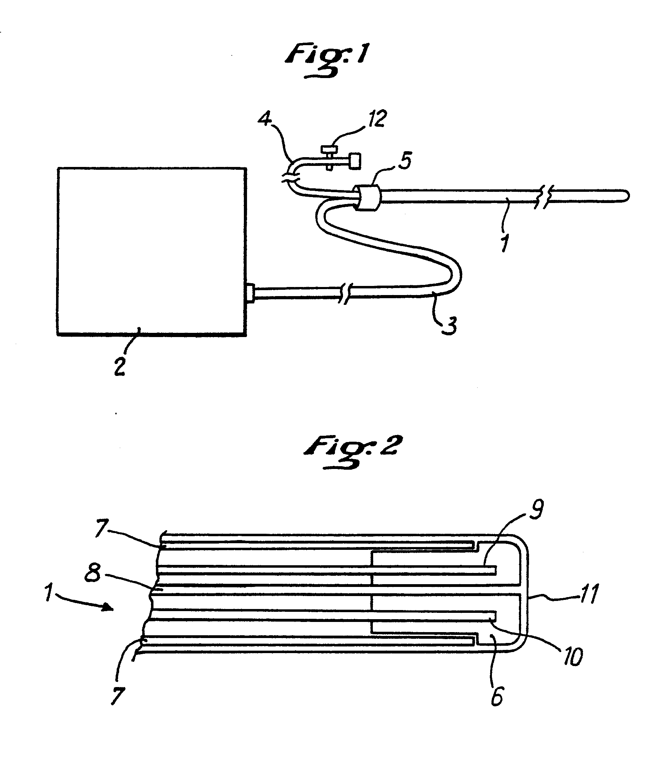

En ce reportant à la figure 1, on peut voir que l'amnioscope selon l'invention se compose d'une sonde 1, d'un appareil de mesure 2, d'un câble à fibres optiques 3 reliant la sonde et l'appareil de mesure, et d'un tuyau 4 susceptible d'être raccordé à une source de liquide aseptique. La sonde 1 a un diamètre d'environ 5mm et une longueur d'environ 30 cm. Son extrémité proximale comporte un connecteur 5 assurant le raccordement au câble 3 et au tuyau 4.

La sonde 1 (Figure 2) présente une extrémité distale constituée d'un embout 6 transparent, souple, et de forme adaptée pour ne pas être traumatisante vis à vis de la poche des eaux. Cet embout 6 est porté par une gaîne tubulaire 7. Au milieu par exemple, de l'embout 6, débouche un canal 8 susceptible d'amener un liquide de lavage de l'extrémité distale de la sonde.

Le canal 8 est relié, dans le connecteur 5, au tuyau 4. Au voisinage de l'extrémité de l'embout 6, et dans le corps de l'embout, aboutissent des fibres optiques : au moins une fibre d'amenée de lumière 9, et au moins une fibre d'observation 10. Ces fibres sont logées dans la gaîne 7, comme le canal 8 d'amenée du liquide de lavage, et elles sont reliées optiquement au câble 3 par l'intermédiaire du connecteur 5.

Autour de la sonde 1 est prévue, de préférence, une enveloppe 11 de protection, facilement déchirable par simple traction sur sa partie voisine du connecteur 5. Cette enveloppe 11 est destinée à être retirée après la mise en place de la sonde, Elle a pour rôle de protéger au cours de la mise en place, l'extrémité distale de la sonde de toute trace de produit susceptible d'affecter soit la transmission optique, soit le résultat de l'observation.

En raison de son faible diamètre, environ 5 mm, la sonde peut être utilisée même lorsque le col utérin est dit "fermé", c'est-à-dire dès le huitième mois de la grossesse. Une fois la sonde 1 mise en place, on procède à l'enlèvement de l'enveloppe déchirable 11. Par l'intermédiaire d'un robinet 12 (Figure 1) on peut assurer un lavage de l'extrémité distale de la sonde au moyen d'un liquide adéquat passant par le canal 8. Il va de soi qu'on peut n'utiliser qu'un seul des moyens de protection de l'extrémité de la sonde : enveloppe extérieure déchirable 11, ou lavage par un liquide amené par le canal 8.

L'appareil de mesure 2 comporte une source de lumière de spectre défini, un analyseur spectroscopique, un affichage et des commandes. Tous ces éléments étant en eux-mêmes connus, ils ne font pas l'objet d'une description. La source de lumière transmet, par la fibre optique 9, une lumière de spectre défini, jusquàau niveau de la poche des eaux.

L'analyseur spectrométrique reçoit, par la fibre 10, la lumière diffusée par le liquide amniotique, et il l'analyse de façon qualitative pour déterminer la présence, dans le liquide amniotique, des colorations caractéristiques de certains corps révélateurs d'une anomalie ou d'un état pathologique du foetus.

La coloration verte est liée à la présence de méconium, la coloration jaune à la présence de bilirubine, et la coloration rouge à la présence d'hémoglobine.

L'analyseur spectrométrique effectue automatiquement une analyse spectrométrique qualitative adaptée aux colorations à reconnaître, c'est-à-dire aux pigments caractéristiques correspondant à ces colorations. Cette analyse est effectuée de manière classique par association de filtres colorés et de détecteurs de lumière.

Elle n'est pas nécessairement quantitative, ce qui permet d'utiliser un analyseur spectrométrique simplifié pour la reconnaissance de certains pigments caractérisés par des longueurs d'onde spécifiques. En raison de cette spécificité, l'analyseur spectrométrique donne un résultat objectif et sûr, plus fiable qu'un examen purement visuel. Le spectre de la lumière émise par la source est défini pour contenir les longueurs d'ondes caractéristiques des pigments recherchés.

Selon la présente invention, la sonde 1 est remplaçable. Elle peut être réutilisable après stérilisation, ou simplement jetable en raison de son faible coût.

Dans la description de la figure 2, on a mentionné une fibre optique 9 d'amenée de lumière et une fibre optique 10 d'observation. Il va de soi que l'amenée de lumière à l'extrémité distale de la sonde peut être assurée par plusieurs fibres 9 de même que la transmission de la lumière diffusée par le liquide amniotique peut être assurée par plusieurs fibres 10.

Pa rapport aux appareils existants, l'amnioscope selon l'invention présente deux avantages importants :
- grâce au faible diamètre de la sonde, il peut être utilisé même lorsque le col utérin est dit "fermé", c'est-à-dire dès le huitième mois de la grossesse ;
- grâce à l'objectivité de la mesure effectuée par l'analyseur spectrométrique, le diagnostic est plus fiable.

**Revendications**

1. - Amnioscope à sonde conductrice de lumière pour l'examen du liquide amniotique inutero, caractérisé en ce qu'il comporte : une sonde (1) remplaçable de faible diamètre contenant des fibres optiques pour la transmission de la lumière d'éclairement du liquide amniotique et de la lumière diffusée par le liquide amniotique ; un appareil de mesure (2) contenant une source de lumière de spectre défini et un analyseur spectroscopique ; et un câble (3) à fibres optiques relié d'une part audit appareil de mesure et d'autre part à un connecteur (5) portant la sonde.

2. - Amnioscope selon la revendication 1, caractérisé en ce que la sonde (1) présente à son extrémité distale un embout (6) transparent et souple, porté par une gaîne (7) tubulaire.

3. - Amnioscope selon la revendication 2, carctérisé en ce que dans la gaîne (7) sont disposées au moins une fibre optique (9) d'amenée de lumière à l'embout (6) et au moins une fibre optique (10) de transmission de la lumière diffusée par le liquide amniotique.

4. - Amnioscope selon la revendication 2, caractérisé en ce que dans la gaîne (7) est disposé un canal (8) débouchant à l'extrémité distale de l'embout (6) et susceptible d'amener du liquide de lavage.

5. - Amnioscope selon la revendication 1, caractérisé en ce que le connecteur (5) portant la sonde (1) est relié par un tuyau (4) à une source de liquide aseptique.

6. - Amnioscope selon la revendication 1, caractérisé en ce que la sonde est recouverte d'une enveloppe (11) de protection, déchirable par traction après mise en place de la sonde.

7. - Amnioscope selon la revendication 1, caractérisé en ce que la source de lumière a un spectre défini pour contenir les longueurs d'onde des pigments dont la présence est recherchée dans le liquide amniotique , et correspondant au méconium, à la bilirubine et à l'hémoglobine.

8. - Amnioscope selon la revendication 1, caractérisé en ce que le diamètre de la sonde (1) est d'environ 5mm, ce qui autorise l'examen du liquide amniotique même lorsque le col utérin est dit "fermé".

*Fig. 1*

*Fig. 2*

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 709 214 (J.R. ROBERTSON) * Résumé; colonne 1, lignes 8-19; colonne 3, lignes 5-14; colonne 3, ligne 50 - colonne 4, ligne 23; colonne 6, lignes 1-10; figure 1 * | 1,3 | A 61 B 1/30 A 61 B 5/00 |
| | --- | | |
| Y | GB-A-2 126 717 (HAMAMATSU PHOTONICS K.K.) * Résumé; page 1, lignes 3-10,100-105; page 1, ligne 125 - page 2, ligne 4; page 2, lignes 88-110; page 3, lignes 32-83; figure 2 * | 1,3 | |
| | --- | | |
| A | US-A-3 136 310 (R.J. MELTZER) * Colonne 1, lignes 8-12; colonne 2, lignes 3-56; figures 1,2 * | 1-3,6,8 | |
| | --- | | |
| A | EP-A-0 184 778 (F.E. SILVERSTEIN et al.) * Résumé; page 8, ligne 5 - page 9, ligne 7; page 10, lignes 29-31; page 11, lignes 22-31; figures 1-4 * | 3-6 | DOMAINES TECHNIQUES RECHERCHES (Int. Cl.4) A 61 B |
| | ----- | | |

Le présent rapport a été établi pour toutes les revendications

| Lieu de la recherche | Date d'achèvement de la recherche | Examinateur |
|---|---|---|
| LA HAYE | 16-03-1988 | FERRIGNO, A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)